Europäisches Patentamt

(19) European Patent Office

Office européen des brevets

(11) Publication number: **0 201 344**
**B1**

## EUROPEAN PATENT SPECIFICATION

(45) Date of publication of the patent specification:
**27.12.89**

(51) Int. Cl.⁴: **A 61 B 17/12**

(21) Application number: **86303536.6**

(22) Date of filling: **09.05.86**

(54) Ligating clip and clip applier.

(30) Priority: **10.05.85 US 733281**

(43) Date of publication of application:
**12.11.86 Bulletin 86/46**

(45) Publication of the grant of the patent:
**27.12.89 Bulletin 89/52**

(84) Designated Contracting States:
**DE FR GB IT SE**

(56) References cited:
**EP-A-0 128 011**
**FR-A-2 062 804**
**FR-A-2 476 478**
**US-A-95 249**
**US-A-3 247 852**

(73) Proprietor: **ETHICON INC., U.S. Route 22,
Somerville New Jersey 08876 (US)**

(72) Inventor: **Transue, James Anthony, 301 E. Main
Street, Somerville, NJ 08876 (US)**
Inventor: **Gertzman, Arthur A., 785 Partridge Drive,
Bridgewater, NJ 08876 (US)**

(74) Representative: **Jones, Alan John, CARPMAELS &
RANSFORD 43 Bloomsbury Square, London,
WC1A 2RA (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convension).

EP 0 201 344 B1

2

## Description

The present invention relates to ligating clips and more particularly to ligating clips fabricated from biocompatible polymeric materials with said clips being of a large size to be used for ligating vascular tissue such as the broad ligament, colonic mesenteries and similar vascular connective tissue.

## Background of the Invention

In many surgical procedures, it is often necessary to ligate a plurality of vessels within the surgical site. The vessels may then be severed downstream of the ligated portion. The primary reason for ligating vessels is to maintain the surgical site free of an excess of blood and to reduce blood loss in the patient. Generally, most of these vessels are individual vessels or vessels that can be readily dissected from adjoining tissue so that a small ligating clip may be placed about the vessel to shut it off. Such clips, which may be either a metal clip or a polymeric clip, are used as a substitute for a ligature or a thread which a surgeon would use to tie around the vessel. The clips substituted for such ligatures, of course reduce the time consumed in shutting off the vessels. Generally, the clips made of metal are narrow U-shaped or V-shaped strips formed of tantalum or stainless steel which are capable of being deformed and possess sufficient strength to retain the deformation of the clip about the vessel. Also in certain instances, the clips are made of a polymeric material, both absorbable and non-absorbable, and are configured so that they have a resilient hinge at one end and some type of interlocking mechanism at the other end. Examples of such metal or plastic clips are shown in U. S. Patents Nos. 3 867 944, 3 631 707, 3 439 523, 3 439 522, 3 363 628, 3 270 745, 4 449 531, and 4 476 865. Generally, the hemostatic or ligating clip is of a small or micro size to keep the foreign material being implanted in the patient as minimal as possible.

In U. S. Patent No. 3 926 195 there is described a plastic clip designed for the temporary and permanent closing of the oviduct and vas deferens in humans. These clips have a clamping surface of from 6 to 10 millimeters in length and from 3 to 6 millimeters in width.

While all of the above-described clips may be used to close individual vessels or tubes, there are certain procedures where the vascular system is so intermeshed in tissue that it is impossible to use individual clips to close off that vascular system. For example, in a hysterectomy, the uterus is partially held in position by vascular connective tissue generally termed the broad ligament. The broad ligament comprises a double fold of tissue which is quite vascular; that is, has a number of vessels carrying blood disposed in or about the broad ligament. The ligament may be from 8 to 10 centimeters in length and a few centimeters in width. In performing a hysterectomy the broad ligaments on each side of the uterus must be tied off or closed, that is, hemostasis must be obtained throughout the vascular system of the broad ligament. In the past, this has generally been accomplished by the surgeon crushing increments of the broad ligament across its width and tying off the crushed portion with an appropriate suture. This can be a time consuming process. This traumatic clamping coupled with the bunched pedicles of tissue could predispose the area to abdominal adhesions. There are a number of other surgical procedures which require hemostasis or the tying off of vascular systems disposed within tissue and in which individual vessels cannot be dissected and ligated but the entire system must be ligated. These are surgical procedures such as an appendectomy, bowel resection, and the like.

From EP-A-0 128 011 there is known a ligating clip for ligating vessels which have not been fully dissected from surrounding tissue. The clip comprises a first leg member and a second leg member, which leg members are connected at their proximal ends by a resilient hinge. Each of the leg members has a tissue clamping surface disposed in opposing relationship to the tissue clamping surface of the other leg member, the tissue clamping surfaces being approximately parallel when the clip is in a closed position.

The first leg member terminates at its distal end in a tissue penetrating means. Said means extends in a direction towards the distal end of the second leg member when the clip is bent about the resilient hinge portion. The distal end of the first leg member has a first locking means and the second leg member terminates at its distal end in a second locking means for interlocking with the first locking means when the clip is in a closed position. The second leg member includes means disposed at its distal end for providing a transverse interlock to prevent lateral movement between the leg members when the clip is in the closed position.

Another embodiment of a ligating clip disclosed in EP-A-0 128 011 differs from the above described embodiment in that the resilient hinge is substituted by a double strap type hinge. The tissue penetrating means is disposed near the distal end of the first leg member and the second leg member includes means disposed near its distal end which provides, as well as the transverse interlock, shrouding of the tissue penetrating means of the first leg member when the clip is in the closed position.

It is an object of the present invention to provide a ligating clip that may be used with vascular systems connected to or running through tissue. It is an object of this invention to provide a flat line of hemostatic pressure without causing pressure necrosis as with metal clamps, traumatic perforations as with staple and precluding bunched pedicles as with ligatures. It is an object of the present invention to provide a ligating clip that may be used without requiring the dissecting of the vessels from the surrounding tissue. It is a further object of the present invention to provide a clip to

2

close off a vascular tissue system which will not irritate or cause trauma to the surrounding tissue. It is a further object of the present invention to provide a clip that has minimal lateral movement of the clamping surfaces once the clip is closed. It is a further object of the present invention to provide a clip which will not interfere with future diagnostic techniques such as x-ray imaging, CT scanning, and the like. It is a further object of the present invention to provide a clip for use in closing vascular tissue which is economical to manufacture. It is a further object of the present invention to provide an instrument for applying such clips to vascular tissue.

**Summary of the Invention**

The present invention provides a sterile, biocompatible clip for use in closing or stopping the blood flow in vascular tissue. The clip has a first leg member and a second leg member with the leg members connected at their proximal ends by a resilient hinge. Each of the leg members has a tissue clamping surface. The clamping surface of one leg member is disposed in opposing relationship to the clamping surface of the other leg member so that the clamping surfaces are approximately parallel when the clip is in the closed position. The clamping surfaces have a length of approximately at least 2 centimeters. The legs are reinforced longitudinally with structural ribbing to minimize material content. The first leg member terminates at its proximal end in a tissue penetrating means which extends in a direction towards the distal end of the second leg member when the clip is bent about the resilient hinge. In the preferred embodiments of the clip of the present invention, the tissue penetrating member comprises a sharp point. The distal end of the first leg member also has a first locking means. The second leg member terminates at its distal end in a second locking means which interlocks with the first locking means when the clip is in the closed position. The second leg member also includes means disposed at its distal end for shrouding the tissue penetrating means of the first leg member when the clip is in the closed position. This shrouding of the tissue penetrating means prevents irritation or trauma that might be caused to adjacent tissue by virtue of the use of the clip. The shroud also prevents lateral movement between the leg member when the clip is in the closed position.

In a preferred embodiment of the present invention, the leg members of the clip are longitudinally reinforced by a ridge disposed longitudinally on the outer surface of the clip opposite the tissue clamping surface. It is desirable to reinforce these legs to be certain that the legs will stay parallel when they are closed about the vascular tissue and will not bend or bow and, hence, not provide hemostasis. The beam faces of the legs contain one and two longitudinal ribs respectively to form the tissue into a labyrinth for further transverse security. Preferably, a central cone point and its receiving recess prevent the clip from pivoting off of an edge plane of tissue.

The present invention also includes an applier for use with an embodiment of the clip of the present invention, in which embodiment the outer surface of the distal end of each leg member has an opening for receiving pins on the applier. The applier of the present invention is a forceps type instrument, with a pair of legs pivotally hinged together. At one end of these legs disposed from the pivot point are means for gripping or grasping the applier to pivot the legs about the pivot point. Disposed at the opposite end of these legs from the pivot point are a pair of jaws for grasping or holding the clip in an open position. Each of the jaws include a pair of longitudinally spaced apart pins extending from one jaw in a direction towards the other jaw. The pins engage the openings disposed at the distal ends of the clip and by virtue of being spaced apart prevent rotation of a clip being held by the jaws.

**Brief Description of the Drawings**

The present invention will be further described in conjunction with the accompanying drawings wherein:

Fig. 1    is a plan view of the clip of the present invention in a fully open position;

Fig. 2    is a bottom view taken at 2 - 2 of Fig. 1;

Fig. 3    is an end view taken at 3 - 3 of Fig. 1;

Fig. 4    is another end view taken at 4 - 4 of Fig. 1;

Fig. 5    is a perspective view of the clip of the present invention in an open position ready for application;

Fig. 6    is a plan view of a clip applier of the present invention; and

Fig. 7    is a plan view of a clip applier of the present invention with a clip placed in the open position in the jaws of the applier.

**Detailed Description of the Drawings**

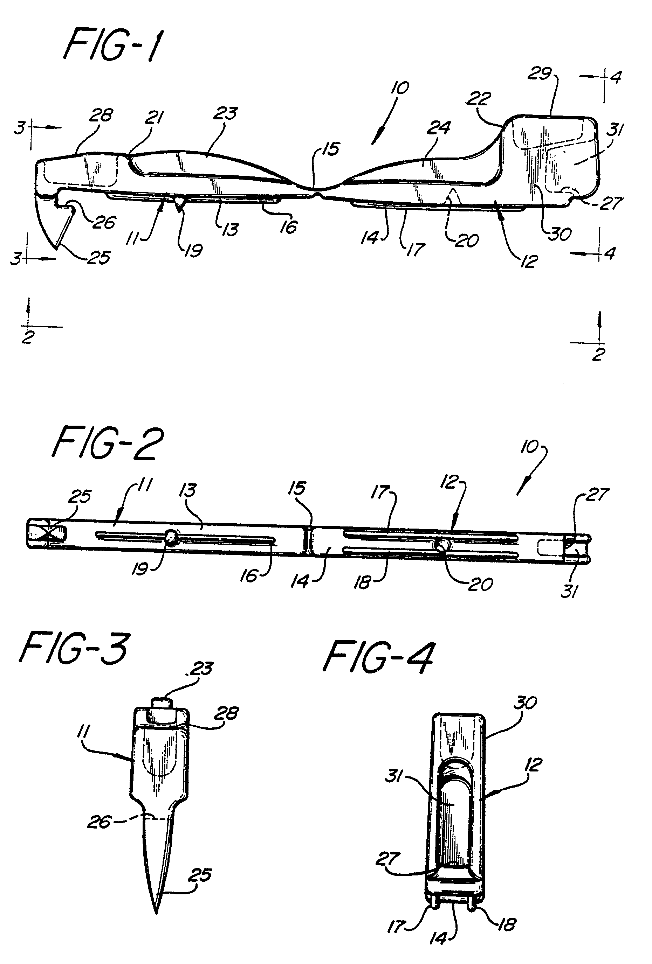

Referring to Figs. 1 through 4 of the drawings, there is shown a clip of the present invention. The clip 10 comprises a first leg member 11 and a second leg member 12. The leg members have tissue clamping surface 13 and 14 respectively. The leg members are joined at the proximal ends by a resilient hinge portion 15. On the tissue clamping surface of one of the leg members there is a longitudinally extending ridge 16 and on the tissue clamping surface of the opposite leg member there are two longitudinally extending ridges 17 and 18. These latter ridges are spaced apart so that when the leg members are brought into a parallel relationship the ridge on the first leg member will position itself between the pair of ridges on the second leg member. Though in this embodiment longitudinal ridges have been shown, the ridges, of course, can be transverse or other configurations as desired to aid in gripping tissue. To also aid in the gripping of the tissue there is disposed or extending from the tissue clamping surface of the first leg member a sharp point 19 and disposed in the tissue clamping surface of the opposite leg member is a corresponding recess 20 so that when the legs are brought into a parallel position the point on the first leg member enters the recess of the second leg member to grip tissue placed between the legs. The sharp point 19 also prevents rotation about the latch on an edge plane of tissue.

Along the back or outer surface 21 and 22 of each member, extending from the resilient hinge portion to the distal end of the leg member, is a narrowed upstanding ridge 23 and 24 which longitudinally reinforces and provides rigidity to the leg member. The structural ribbing provides rigidity while minimizing material content. Because of the size of the clip, it is important that the leg members be reinforced so they remain straight and will not bow or bend or otherwise deform during use. Disposed at the distal end of the first leg member is a penetrating means 25. The penetrating means is a sharp pointed portion which extends away from the tissue clamping surface of the first leg member and towards the tissue clamping surface of the second leg member when the clip is in the closed position. This sharp pointed portion has an undercut ledge 26 which is the locking means and which will interlock with an appropriate ridge 27 disposed at the distal end of the second leg member. At the back or outer surface of the end leg member and at the distal end thereof is a recess 28 and 29 for accepting an applier gripper means from a suitable applier used to apply the clip. The distal end of the second leg member comprises an expanded portion 30 which has its free end open 31 for accepting the pointed penetrating point of the distal end of the opposite leg member. This opening includes the ridge 27 for interlocking with the ridge 26 at the end of the pointed penetrating means.

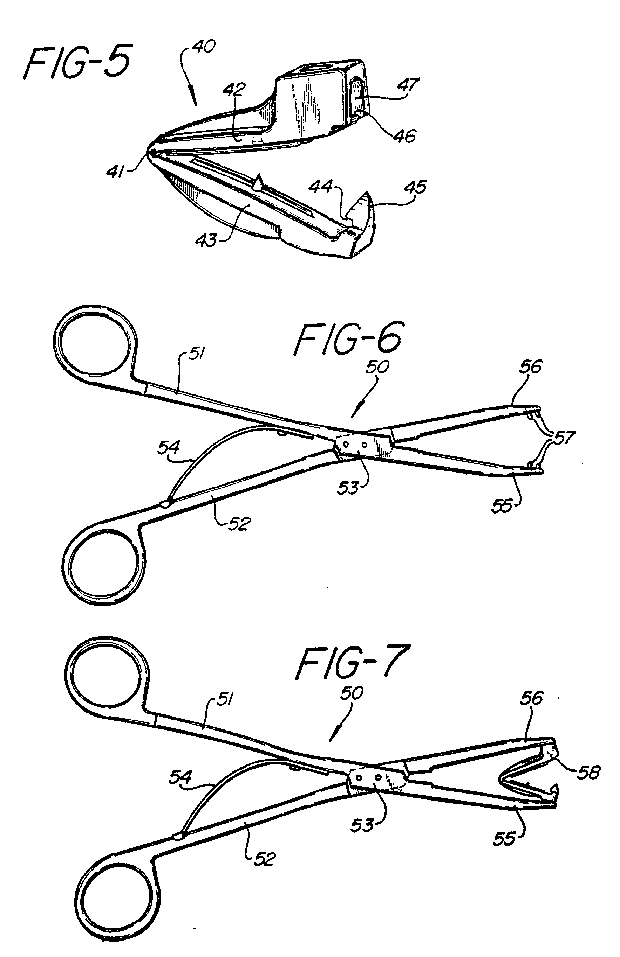

It is a simple matter to mold or produce the clip in the fully opened position as shown in Fig. 1. This may be accomplished by any of the molding techniques as are wellknown in the art. As shown in Fig. 5, the clip 40 is bent about the resilient hinge portion 41 into the configuration as shown in Fig. 5 with the legs 41 and 43 diverging from the resilient hinge while the clip is in the open position. On closing of the clip, the ledge 44 at the bottom of the penetrating point 45 on the first leg member interlocks with the ridge 46 in the opening 47 at the distal end of the second leg member to lock the two legs together and hold the legs in an approximately parallel position. In the closed position the penetrating point is entirely shrouded or enclosed by the distal end of the second leg member so that the point cannot irritate or cause trauma to adjacent tissue and so there is no lateral movement between the leg members 42 and 43.

The clips of the present invention are most conveniently molded of biologically acceptable non-metallic materials which may be absorbable or non-absorbable. Preferred absorbable polymers include homopolymers and copolymers of glycolide and lactide and p-dioxanone. Preferred non-absorbable polymers include nylon and polypropylene.

The tissue clamping surfaces of the clips should be approximately 2 centimeters in length and may be as much as 2 - 1/2 to 3 or even more centimeters in length. This length is important in order to take a substantial bite in the vascular tissue which is to be closed. A plurality of these clips may be used to close a very wide vascular tissue, in doing so the clips are placed incrementally across the width of the vascular tissue to be closed.

Fig. 6 illustrates a forceps-type clip applier comprising two handle members 51 and 52 crossing at hinge point 53 and maintained in a normally opened position by a spring 54. One of the handles extends beyond the hinge forming a jaw member 55 while the extension of the other handle extends beyond the hinge forming a complementary jaw member 56. The jaws are of identical design and each are provided with a pair of longitudinally spaced apart pins 57. The pins extend towards each other as shown. These pins fit in the opening at the back of the distal ends of each of the leg members of the clip when the clip 58 is placed in the instrument in the open position as shown in Fig. 7.

The longitudinally spaced apart pins hold the clip in the applier and aid in preventing rotation of the clip in the applier. Also, by placing these pins directly over the distal end portions of the clip, the force of the applier is directly applied to the portion of the clip which is used to penetrate the tissue. The applier allows for total visibility of the clip during use and is especially suitable for use in surgical procedures where visibility is at a premium. The resilient hinge portion of the clip which tends to force the legs to diverge aids in holding the clip in the applier by pressing the distal ends of the clip against the jaws of the applier.

As can be appreciated, many variations in both the clip design and the applier design other than the embodiments disclosed herein will be apparent to those skilled in the art and are contemplated within the scope of the present invention.

Having now described the invention in considerable detail, it should be readily apparent to those skilled in the art that various modifications and alterations may be made to the invention without departing from the scope thereof.

## Claims

1. A ligating clip (10) for ligating vascular tissue, said clip (10) comprising a first leg member (11) and a second leg member (12), said leg members (11, 12) being connected at their proximal ends by a resilient hinge (15), each of said leg members (11, 12) having a tissue clamping surface (13, 14) disposed in opposing relationship to the tissue clamping surface (13, 14) of the other leg member (12, 11), said tissue clamping surface (13, 14) being ap,proximately parallel when said clip (10) is in a closed position, each of said tissue clamping surface (13, 14) having a length of approximately at least 2 centimeters, each of said leg members (11, 12) having longitudinally disposed reinforcing means (23, 24) said first leg member (11) terminating at its distal end in a tissue penetrating means (25), said means (25) extending in a direction towards the distal end of the second leg member (12) when the clip is bent about the resilient hinge, the distal end of said first leg member (11) having a first locking means (26) the second leg member (12) terminating at its distal end in a second locking means (27) interlocking with said first locking means (26) when said clip (10) is in a closed position, and said second leg member (12) including means (30) disposed at its distal end for shrouding the tissue penetrating means (25) of said first leg member (11) when the clip (10) is in the closed position and providing a transverse interlock to prevent lateral movement between the leg members (11, 12) when the clip (10) is in the closed position.

2. A clip (10) according to Claim 1 wherein the tissue penetrating means comprises a sharp point (25) extending from said first leg member (11) towards said second leg member (12) when the clip is bent about the resilient hinge.

3. A clip (10) according to Claim 2 wherein the locking means of said first leg member (11) comprises an undercut ledge (26) at the base of said sharp point (25) and the locking means on said second leg member (12) comprises a shoulder (27) disposed at the distal end of said leg member (12) which interengages with the undercut ledge (26) on the sharp point (25) to interlock the leg members (11, 12) when the clip (10) is in the closed position.

4. A clip according to any one of Claims 1 to 3 wherein the resilient hinge comprises a thinned area (15) joining the first and second leg members.

5. A clip according to any one of Claims 1 to 4 wherein the tissue clamping surface (13) of the first leg member (11) has a longitudinal ridge (16) disposed in the center of said tissue clamping surface (13) and the tissue clamping surface of the second leg member (12) has a pair of spaced apart longitudinally extending ridges (17, 18) disposed thereon so that when the clip (10) is closed the ridge on the first leg member (11) is disposed between the ridges (17, 18) on the second leg member (12).

6. A clip according to any one of Claims 1 to 5 wherein the tissue clamping surface (13) of the first leg member (11) has a centrally located sharpened point (19) extending from said surface (13), the tissue clamping surface (14) of the second leg member (12) has a recess (20) disposed in the clamping surface (14) in which the point (19) is disposed when the clip (10) is in the closed position.

7. A clip according to any one of Claims 1 to 6 wherein the longitudinally disposed reinforcing means comprises a longitudinally disposed ridge (23, 24) on the back surface of each leg member (11, 12), said longitudinally extending ridge (23, 24) being narrower than the body of the leg member (11, 12).

8. A clip according to any one of Claims 1 to 7 wherein the outer surface of the distal end of each leg member (11, 12) has an opening (28, 29) disposed in said outer surface for accepting means on a suitable instrument (50) for holding the clip (10) in said instrument (50).

9. An instrument (50), for applying a ligating clip (10, 40, 58) according to Claim 8, said instrument comprising a pair of handles (51, 52) pivoted about a hinge (53) point, said handles crossing at said hinge point and extending beyond the hinge point to form a pair of clip closing jaws (55, 56) having opposed inner faces, characterised in that each of said inner faces has a pair of longitudinally spaced apart pins (57) extending towards the other jaw for engaging with said openings (28, 29) in the distal end of one of said leg members (11, 12) of the clip to be applied by the instrument.

**Patentansprüche**

1. Ligaturklammer (10) zum Abbinden von Gefäßgewebe, wobei die genannte Klammer (10) umfaßt: einen ersten Schenkel (11) und einen zweiten Schenkel (12), wobei die genannten Schenkel (11, 12) an ihren hinteren Enden durch ein elastisches Scharnier (15) verbunden sind, eine Gewebeklemmfläche (13, 14) an jedem der genannten Schenkel (11, 12), die der Gewebeklemmfläche (14, 13) des anderen Schenkels (11, 12) gegenüberliegt, wobei die Gewebeklemmflächen (13, 14) annähernd parallel verlaufen, wenn die genannte Klammer (10) geschlossen ist, eine Länge jeder der genannten Gewebeklemmflächen (13, 14) von annähernd mindestens 2 cm, in Längsrichtung angeordnete Verstärkungsmittel (23, 24) an jedem der Schenkel (11, 12), ein das Gewebe durchdringendes Mittel (25) am vorderen Ende des genannten ersten Schenkels (11), wobei das genannte Mittel (25) sich in einer Richtung zu dem vorderen Ende des zweiten Schenkels (12) erstreckt, wenn die Klammer um das elastische Scharnier gebogen ist, eine erste Verriegelungsvorrichtung (26) am vorderen Ende des genannten ersten Schenkels (11), eine zweite Verriegelungsvorrichtung (27) am vorderen Ende des zweiten Schenkels (12) zur Verriegelung mit der genannten ersten Verriegelungsvorrichtung (26), wenn die genannte Klammer (10) geschlossen ist, und eine Vorrichtung (30) am genannten zweiten Schenkel (12), die an seinem vorderen Ende zum Abschirmen des das Gewebe durchdringenden Mittels (25) des ersten Schenkels (11) angeordnet ist, wenn die Klammer (10) geschlossen ist, und eine quer gerichtete Verriegelung vorsieht, um eine seitliche Bewegung zwischen den Schenkeln (11, 12) zu verhindern, wenn die Klammer (10) geschlossen ist.

2. Klammer nach Anspruch 1, bei der das das Gewebe durchdringende Mittel aus einer scharfen Spitze (25) besteht, die sich von dem genannten ersten Schenkel (11) zu dem genannten zweiten Schenkel (12) erstreckt, wenn die Klammer um das elastische Scharnier gebogen ist.

3. Klammer (10) nach Anspruch 2, bei der die Verriegelungsvorrichtung des genannten ersten Schenkels (11) aus einem hinterschnittenen Vorsprung (26) an der Basis der genannten scharfen Spitze (25) besteht und die Verriegelungsvorrichtung an dem genannten zweiten Schenkel (12) eine Schulter (27) umfaßt, die an dem vorderen Ende des genannten Schenkels (12) angeordnet ist und mit dem hinterschnittenen Vorsprung (26) an der scharfen Spitze (25) zusammenwirkt, um die Schenkel (11, 12) miteinander zu verriegeln, wenn die Klammer (10) geschlossen ist.

4. Klammer nach einem der Ansprüche 1 bis 3, bei der das elastische Scharnier einen verdünnten Bereich (15) umfaßt, der die ersten und zweiten Schenkel miteinander verbindet.

5. Klammer nach einem der Ansprüche 1 bis 4, bei der die Gewebeklemmfläche (13) des ersten Schenkels (11) eine Längsrippe (16) aufweist, die in der Mitte der Gewebeklemmfläche (13) angeordnet ist, und die Gewebeklemmfläche des zweiten Schenkels (12) ein Paar von im Abstand von einander angeordneten, sich längserstreckenden Rippen (17, 18) aufweist, die darauf derart angeordnet sind, daß, wenn die Klammer (10) geschlossen ist, die Rippe (16) auf dem ersten Schenkel (11) zwischen den Rippen (17, 18) auf dem zweiten Schenkel (12) angeordnet ist.

6. Klammer nach einem der Ansprüche 1 bis 5, bei der die Gewebeklemmfläche (13) des ersten Schenkels (11) eine mittig angeordnete geschärfte Spitze (19) hat, die sich von der genannten Fläche (13) erstreckt, wobei die Gewebeklemmfläche (14) des zweiten Schenkels (12) eine in der Klemmfläche (14) angeordnete Ausnehmung (20) aufweist, in welcher die Spitze (19) angeordnet ist, wenn die Klammer (10) geschlossen ist.

7. Klammer nach einem der Ansprüche 1 bis 6, bei der die in Längsrichtung angeordnete Verstärkungsvorrichtung aus einer in Längsrichtung angeordneten Rippe (23, 24) auf der Rückseite jedes Schenkels (11, 12) besteht, wobei die genannte, sich längserstreckende Rippe (23, 24) schmaler als der Körper der Schenkel (11, 12) ausgebildet ist.

8. Klammer nach einem der Ansprüche 1 bis 7, bei der die Außenseite des vorderen Endes jedes Schenkels (11, 12) eine Öffnung (28, 29) hat, die in den genannten Außenseiten zur Aufnahme von Mitteln an einem geeigneten Instrument (50) zur Halterung der Klammer (10) in dem genannten Instrument (50) angeordnet ist.

9. Instrument (50) zum Anbringen einer Ligaturklammer (10, 40, 58) gemäß Anspruch 8, wobei das genannte Instrument ein Paar Griffe (51, 52) umfaßt, die um einen Scharnierpunkt (53) schwenkbar sind, wobei die Griffe sich an dem genannten Scharnierpunkt kreuzen und sich über den Scharnierpunkt zur Bildung eines Paares Klammerschließbacken (55, 56) mit sich gegenüberliegenden Innenseiten hinaus erstrecken, dadurch gekennzeichnet, daß jede der genannten Innenseiten ein Paar von sich in Längsrichtung beabstandeten Stiften (57) aufweist, die sich in Richtung der anderen Backe zum Eingriff in die genannten Öffnungen (28, 29) im vorderen Ende eines der genannten Schenkel (11, 12) der durch das Instrument anzubringenden Klammer erstrecken.

**Revendications**

1. Pince de ligature (10) pour ligaturer un tissu vasculaire, ladite pince (10) comprenant un premier élément de bras (11) et un second élément de bras (12), lesdits éléments de bras (11, 12) étant reliés à leurs extrémités proximales par une charnière élastique (15), chacun desdits éléments de bras (11, 12) ayant une surface de pincement des tissus (13, 14) disposée en une relation opposée avec la surface de pincement des tissus (14, 13) de l'autre élément de bras (12, 11), lesdites surfaces de pincement des tissus (13, 14) étant approximativement parallèles lorsque ladite pince (17) est dans une position fermée, chacune desdites surfaces de pincement des tissus (13, 14) ayant une longueur d'environ au moins 2 cm, chacun desdits éléments de bras (11, 12) ayant des moyens de renforcement disposés longitudinalement (23, 24), ledit premier élément de bras se terminant à son extrémité distale dans un moyen de pénétration des tissus (25), ledit moyen (25) s'étendant dans une direction allant vers l'extrémité distale du second élément de bras (12) lorsque la pince est pliée autour de la charnière élastique, l'extrémité distale dudit premier élément de bras (11) ayant un premier moyen de blocage (26), le second élément de bras (12) se terminant à son extrémité distale dans un second moyen de blocage (27) pour s'enclencher avec ledit premier moyen de blocage (26) lorsque ladite pince (10) est en position fermée et ledit second élément de bras (12) incluant un second moyen (30) disposé à son extrémité distale pour envelopper le moyen de pénétration des tissus (25) dudit premier élément de bras (11) lorsque la pince (10) est en position fermée et assurant un enclenchement transversal pour empêcher tout mouvement latéral entre les éléments de bras (11, 12) lorsque la pince (10) est en position fermée.

2. Pince (10) selon la revendication 1 dans laquelle le moyen de pénétration des tissus comprend une pointe aiguë (25) s'étendant à partir du premier élément de bras (11) vers ledit second élément de bras (12) lorsque la pince est pliée autour de la charnière élastique.

3. Pince (10) selon la revendication 2, dans laquelle le moyen de blocage dudit premier élément de bras (11) comprend un rebord en encoche (26) à la base de ladite pointe aiguë (25), et le moyen de blocage sur ledit second élément de bras (12) comprend un épaulement (27) disposé à l'extrémité distale dudit élément de bras (12) qui s'inter-engage avec le rebord en encoche (26) sur la pointe aiguë (25) pour enclencher les éléments de bras (11, 12) lorsque la pince (10) est en position fermée.

4. Pince selon l'une quelconque des revendication 1 à 3 dans laquelle la charnière élastique comprend une zone amincie (15) adjoignant les premier et second éléments de bras.

5. Pince selon l'une quelconque des revendications 1 à 4, dans laquelle la surface de pincement des tissus (13) du premier élément de bras (11) a une crête longitudinale (16) disposée au centre de ladite surface de pincement des tissus (13) et la surface de pincement des tissus du second élément de bras a une paire de crêtes espacées longitudinalement (17, 18) qui y sont disposées de manière que lorsque la pince (10) est fermée, la crête sur le premier élément de bras (11) est disposée entre les crêtes (17, 18) sur le second élément de bras (12).

6. Pince selon l'une quelconque des revendications 1 à 5 dans laquelle la surface de pincement des tissus (13) du premier élément de bras (11) présente une pointe aiguë située en position centrale (19), s'étendant à partir de ladite surface (13), la surface de pincement des tissus (14) du second élément de bras (12) présente un évidement (20) disposé dans la surface de pincement (14) où la pointe (19) est disposée lorsque la pince (10) est en position fermée.

7. Pince selon l'une quelconque des revendications 1 à 6, dans laquelle le moyen de renforcement disposé longitudinalement comprend une crête disposée longitudinalement (23, 24) à la surface arrière de chaque élément de bras (11, 12) ladite crête s'étendant longitudinalement (23, 24) étant plus étroite que le corps de l'élément de bras (11, 12).

8. Pince selon l'une quelconque des revendications 1 à 7 dans laquelle la surface extérieure de l'extrémité distale de chaque élément de bras (11, 12) présente une ouverture (28, 29) disposée dans ladite surface externe pour recevoir un moyen prévu sur un instrument approprié (50) afin de maintenir la pince (10) dans ledit instrument (50).

9. Instrument (50) pour appliquer une pince de ligature (10, 40, 58) selon la revendication 8, ledit instrument comprenant une paire de poignées (51, 52) articulé autour d'un point charnière (53), lesdites poignées se croisant audit point charnière et s'étendant au-delà dudit point charnière pour former une paire de mâchoires (55, 56) fermant la pince et ayant des faces internes opposées, caractérisé en ce que chacune desdites faces internes a une paire de broches espacées longitudinalement (57) s'étendant vers l'autre mâchoire afin de s'engager avec lesdites ouvertures (28, 29) dans l'extrémité distale d'un desdits éléments de bras (11, 12) de la pince à appliquer par l'instrument.

FIG-1

FIG-2

FIG-3

FIG-4

FIG-5

FIG-6

FIG-7